# EUROPEAN PATENT APPLICATION

(11) **EP 2 505 202 A1**
(43) Date of publication of application: **03.10.2012**
(21) Application number: 11002649.9
(22) Date of filing: 30.03.2011
(51) Int. Cl.: A61K 31/6615, A61P 7/02, A61P 9/10

(54) **D-myo-inositol 3,4,5,6-tetrakisphosphate to modulate thrombin/antithrombin activity by heparin binding**

(71) Applicant: Karlsruher Institut Für Technologie (KIT), 76131 Karlsruhe (DE); Universidad De Murcia, 30003 Murcia (ES)
(72) Inventor: Péréz-Sánchez, Horacio, 76185 Karlsruhe (DE); Meliciani, Irene, 76137 Karlsruhe (DE); Wenzel, Wolfgang, 76356 Weingarten (DE); Martinez-Martinez, Irene, 30108 Murcia (ES); Navarro-Fernández, José, 30108 Murcia (ES); De La Calle, Javier Corral, 30506 Murcia (ES); Garciá, Vicente Vicente, 30009 Murcia (ES)
(74) Representative: Müller-Boré & Partner Patentanwälte

(57) **Abstract**

The present invention relates to D-*myo*-inositol 3,4,5,6-tetrakisphosphate for use in activating antithrombin in a patient as well as to a pharmaceutical composition containing D-*myo*-inositol 3,4,5,6-tetrakisphosphate for use in recovery of antithrombin activity in patients with antithrombin deficiency or in prophylaxis for a disease associated with increased blood clotting.

## Description

The present invention relates to D-*myo*-inositol 3,4,5,6-tetrakisphosphate for use in activating antithrombin in a patient as well as to a pharmaceutical composition containing D-*myo*-inositol 3,4,5,6-tetrakisphosphate for use in recovery of antithrombin activity in patients with antithrombin deficiency or in prophylaxis for a disease associated with increased blood clotting.

The closed and high pressured human circulatory system requires a potent and rapid hemostatic system to avoid otherwise lethal consequences of endothelial damage. An injury of blood vessel or even mild impairments of blood coagulation system may cause hemorrhage or formation of blood clot, or thrombus, which may lead to a risk of serious diseases such as hemophilia or thromboembolic diseases. Particularly, thromboembolic diseases such as stroke and myocardial infarction are the leading cause of mortality and disability worldwide, and thus development of effective treatment and prophylaxis for these diseases are a major concern in pharmaceutical development.

Blood coagulation is regulated by a complex cascade of various coagulation factors. Among the coagulation factors, thrombin plays a major role in the blood coagulation cascade, regulating the amount of blood clotting. Thrombin is a serine protease which catalyzes the transformation of fibrinogen to fibrin, forming a fibrin clot. Thrombin is also responsible for activation of other blood-coagulating factors, such as factor V, factor VIII, factor XI, and factor XIII.

However, an uncontrolled excess of thrombin can result in thrombotic episodes associated with serious diseases and even death. Therefore, regulation of thrombin by specific inactivation is necessary in order to maintain the proper function of the circulatory system. Antithrombin is the main regulator of thrombin by a strong and efficient mechanism of inhibition shared by serpins by covalently binding to thrombin. Therefore, antithrombin has been an important therapeutic target for prophylaxis and treatment of diseases associated with blood clotting.

Already existing compounds that can activate antithrombin are based on sugars, mainly heparin. Heparin may cause heparin-induced thrombocytopenia (HIT) in patients. Since heparin is only effective in the presence of antithrombin, the efficacy of heparin can be low if a patient has a low level of antithrombin activity. Other sugar-based antithrombin activators such as low molecular weight heparin (LMWH) and fondaparinux are mainly excreted in kidneys, and thus they can cause bleeding complications when administered to patients having kidney deficiency. The sugar-based antithrombin activators also have undesirable side effects, and in some cases they cannot be administrated to patients with sensitivity to sugars. In addition, sugars are usually very difficult to synthesize and therefore expensive, which limit their applicability as drugs.

Other hemostatic elements have been also targets of common treatments for thrombosis and thromboembolism. Known drugs include antiplatelets (aspirin, clopidogrel, or anti-IIb/IIIa), anticoagulants (acenocoumarol, warfarin, anti-II, or anti-FXa) and fibrinolytic drugs.

In the patent application JP1986-51325, a chemical synthesis of *myo*-inositol 3,4,5,6-tetrakisphosphate is described. However, *myo*-inositol 3,4,5,6-tetrakisphosphate has not been used as a therapeutic to prevent or treat diseases associated with blood clotting.

Thus, the problem underlying the present invention is to provide a novel compound for use in an effective therapy or prophylaxis for diseases associated with blood clotting in patients.

The solution to the above technical problem is achieved by the embodiments characterized in the claims.

In particular, the present invention relates to D-*myo*-inositol 3,4,5,6-tetrakisphoshate (hereinafter referred to as Ins(3,4,5,6)P₄) for use in activating antithrombin in a patient.

In a preferred embodiment of the present invention, antithrombin is antithrombin III.

In one embodiment, D-*myo*-inositol 3,4,5,6-tetrakisphoshate (hereinafter referred to as Ins(3,4,5,6)P₄) is used in activating antithrombin in a sample from a patient.

The "sample" according to the present invention may be any sample derived from a naturally occurring system, preferably a sample containing a body fluid or components derived from a body fluid. In one embodiment of the present invention, the sample may be a naturally occurring system such as a solution selected from the group consisting of serum, saliva, urine, bile, lymph, tissue, like e.g. bladder or kidney, cerebrospinal fluid (CSF) and/or other body fluids. In a preferred embodiment of the present invention, the sample comprises plasma.

Further, the sample may comprise a solution derived from naturally occurring systems, e.g. a solution containing isolated body fluid compounds or processed body fluids. In another embodiment of the present invention, the sample may comprise cells or tissue samples obtained from a mammal. Methods for obtaining the above samples are known in the prior art.

The sample may be derived from a mammal, preferably a mammal selected from the group consisting of human, mouse, rat, pig, cat, dog, horse, goat, cattle, cow, and monkey and/or others. In a preferred embodiment of the present invention, the sample is derived from a human. In another embodiment of the present invention, the sample contains isolated body fluid compounds or processed body fluids derived from a mammal, preferably a mammal selected from the group consisting of human, mouse, rat, pig, cat, dog, horse, goat, cattle, cow, and monkey and/or others. In a preferred embodiment of the present invention, the sample contains isolated body fluid compounds or processed body fluids derived from a human.

According to the present invention, "patient" can be any mammal, preferably selected from the group consisting of human, mouse, rat, pig, cat, dog, horse, goat, cattle, cow, and monkey and/or others. Most preferably, the patient is a human being. In another preferred embodiment, the patient is a patient having an antithrombin deficiency. In a more preferred embodiment, the patient is a patient having an antithrombin III deficiency.

In one preferred embodiment, the patient has an inherited antithrombin deficiency, more preferably a type I antithrombin deficiency or a type II antithrombin deficiency. In an even more preferred embodiment, the patient is a patient having an Budapest III antithrombin deficiency, wherein antithrombin has a L99F mutation. In another preferred embodiment, the patient has an acquired antithrombin deficiency.

In a more preferred embodiment, the patient has a hypersensitivity to sugars. In an even more preferred embodiment, the patient has diabetes mellitus, most preferably type I diabetes or type II diabetes.

The Ins(3,4,5,6)P₄ according to the present invention may preferably be selected from the group consisting of 1D-*myo*-inositol 3,4,5,6-tetrakisphosphate, 1D-*myo*-inositol 3,4,5,6-tetrakisphosphoric acid, and D-*myo*-inositol 3,4,5,6-tetrakis(dihydrogen phosphate).

The dosage of Ins(3,4,5,6)P₄ may depend on the disease of a patient, e.g. a patient with antithrombin deficiency or a patient with atherosclerosis. Further, the dosage of Ins(3,4,5,6)P₄ for treatment of diseases as defined herein may be different from the dosage of Ins(3,4,5,6)P₄ for prophylaxis. The suitable dossage may be determined by a person skilled in the art.

In a preferred embodiment of the present invention, Ins(3,4,5,6)P₄ is used in activating antithrombin in a patient, wherein the patient is suffering from a disease associated with increased blood clotting.

In one embodiment of the present invention, Ins(3,4,5,6)P₄ is used in activating antithrombin in a sample from a patient, wherein the patient is suffering from a disease associated with increased blood clotting.

In a more preferred embodiment of the present invention, Ins(3,4,5,6)P₄ is used in activating antithrombin in a patient, wherein the patient is suffering from a thromboembolic disease.

In a even more preferred embodiment of the present invention, Ins(3,4,5,6)P₄ is used in activating antithrombin in a patient, wherein the patient is suffering from a thromboembolic disease, wherein the thromboembolic disease is selected from the group consisting of arterial thrombosis, stroke, and myocardial infarction.

According to the present invention, the term "disease" includes any disease caused by increased blood clotting. In a preferred embodiment, the "disease associated with increased blood clotting" is a thrombosis, embolism, or thromboembolic disease. In a preferred embodiment, thrombosis is a formation of a thrombus, or generally called blood clot, which prevents blood from flowing partially or entirely in a blood vessel. In a more preferred embodiment, the disease is anoxia, hypoxia, or ischemia preferably in the tissue located at downstream of an occluded blood vessel. Most preferably, the occlusion is caused by a thrombus. In a preferred embodiment, thrombosis is caused by congenital thrombophilia. In another preferred embodiment, thrombosis is caused by acquired factors, such as cancer, obesity, hypertension, smoking, metabolic syndrome and/or surgery. In another preferred embodiment, thrombosis is caused by arteriosclerosis, more preferably selected from the group consisting of atherosclerosis, arteriolosclerosis, arteriosclerosis obliterans and medial calcific sclerosis. In another preferred embodiment, thrombosis is caused by immobility, such as economy class syndrome.

Thrombosis as used herein is arterial or venous thrombosis. In a preferred embodiment, the disease as defined herein is caused by arterial thrombosis, more preferably a disease selected from the group consisting of stroke, myocardial infarction, or acute leg ischemia. In another preferred embodiment, the disease as defined herein is caused by a venous thrombosis, more preferably selected from the group consisting of deep vein thrombosis, pulmonary embolism, cerebral venous thrombosis, inferior vena cava venous thrombosis, hepatic venous thrombosis, portal venous thrombosis, renal venous thrombosis, axillary venous thrombosis, branchial venous thrombosis, mesenteric venous thrombosis, pelvic venous thrombosis, and retinal venous thrombosis.

In a preferred embodiment, the disease as defined herein is caused by a thrombus which is freed from where it was formed and migrates to another position in a blood vessel, more preferably an embolism. Embolism is a common complication of thrombosis, and the combination of thrombosis and embolism is defined as thromboembolic disease. In a preferred embodiment, the disease as defined herein is a thromboembolic disease, more preferably any one selected from a group consisting of arterial thrombosis, myocardial infarction and stroke.

In another preferred embodiment of the present invention, Ins(3,4,5,6)P₄ is used in activating antithrombin in a patient, wherein the administration of Ins(3,4,5,6)P₄ is a prophylaxis for a disease associated with increased blood clotting.

In another preferred embodiment of the present invention, Ins(3,4,5,6)P₄ is used in activating antithrombin in a patient, wherein Ins(3,4,5,6)P₄ is used in recovery of antithrombin activity in patients with antithrombin deficiency.

According to the present invention, Ins(3,4,5,6)P₄ may be administered by any administration route known in the art being suitable. The route of administration does not exhibit particular limitations and includes for example systemic administration, e.g. topical administration, oral administration, and administration via the peritoneal route, intravenous route, intramuscular route, or subcutaneous route. Optionally, suitable carriers may be used for administration. Ins(3,4,5,6)P₄ may be administered in any form known in the art, e.g. as a liquid, a powder, an aerosol, a capsule, or a tablet.

According to the present invention, "prophylaxis" may be any administration of Ins(3,4,5,6)P₄ to a patient with normal or higher level of blood clotting. In a preferred embodiment, prophylaxis includes the use of Ins(3,4,5,6)P₄ to prevent a disease as defined herein. In another preferred embodiment, prophylaxis includes the use of Ins(3,4,5,6)P₄ in order to prevent blood from clotting, preferably before, during, or after surgery including orthopedic surgery, cardiac surgery, angioplasty, organ transplant, or during dialysis in patients having deficiencies in kidney function.

Additionally, the present invention relates to a pharmaceutical composition containing Ins(3,4,5,6)P₄ for use in recovery of antithrombin activity in patients with antithrombin deficiency.

The "pharmaceutical composition" may further comprise an auxiliary agent, e.g. selected from the group consisting of a pharmaceutically acceptable carrier, diluent, salt, buffer, and excipient. Said pharmaceutical composition can be used for treating the above-defined diseases associated with blood clotting. Further, the pharmaceutical composition may be administered by any route known in the prior art. In one example, the pharmaceutical composition may be administered via oral administration, topical administration, intravenous route, intramuscular route, peritoneal route, or subcontaneous route.

The term "patient with antithrombin deficiency" means a patient having lower antithrombin activity compared to the average thrombin activity of healthy persons, or a patient having no antithrombin activity. A patient having low or no antithrombin activity is preferably a patient having innate or acquired deficiency in antithrombin activity, any patient with low level of antithrombin who suffers from hepatic dysfunction, serious burn, nephrotic syndrome, and/or sepsis. Alternatively, a patient with a low level of antithrombin is preferably a patient under pregnancy. Patients can also be patients taking estrogen or L-asparaginase as medication. Antithrombin deficiency is preferably selected from a group consisting of antithrombin deficiency I and antithrombin deficiency II. In a more preferred embodiment, the patient is a patient having an Budapest III antithrombin deficiency, wherein antithrombin has a L99F mutation.

The present invention further relates to a pharmaceutical composition containing Ins(3,4,5,6)P₄ for use in prophylaxis of a disease associated with increased blood clotting.

The present invention also relates to a method of treatment, wherein Ins(3,4,5,6)P₄ is used for activating antithrombin in a patient. In a preferred embodiment, said patient is a patient suffering from a disease associated with blood clotting as defined herein. In a preferred embodiment, the method of treatment according to the present invention comprises the step of administering Ins(3,4,5,6)P₄ to a patient.

The present invention further relates to a method of prophylaxis, wherein Ins(3,4,5,6)P₄ is used for activating antithrombin in a patient. In a preferred embodiment, said patient is a patient suffering from a disease associated with blood clotting as defined herein. In a preferred embodiment, the method of prophylaxis according to the present invention comprises the step of administering Ins(3,4,5,6)P₄ to a patient.

Ins(3,4,5,6)P₄ activates antithrombin activity of thrombin inhibition by increasing the affinity of antithrombin to heparin. The affinity of heparin to antithrombin can be measured using any standard fluorescence spectroscopic apparatus. Antithrombin contains four tryptophanes that are excited at 280 nm and produce the emission of fluorescence at 340 nm (W49, 189, 225, and 307). When heparin binds to antithrombin, antithrombin changes its conformation and is activated, causing an increase in the intrinsic fluorescence of the protein. Since the fluorescence intensity of antithrombin increases proportionally to the amount of heparin bound, the affinity of antithrombin for the heparin can be calculated indirectly by measuring the emission of fluorescence in the presence of different concentrations of heparin until the affinity reaches saturation.

In a preferred embodiment, Ins(3,4,5,6)P₄ binds to antithrombin with an affinity constant of Kd = 45 nM, which is higher than an affinity of pentasaccharide Fondaparinux (Arixtra ®) to antithrombin (Kd = 273 nM). In another preferred embodiment, calorimetry shows a ΔH (-1215 cal/mol) of Ins(3,4,5,6)P₄, which is significantly less than that of heparin pentasaccharide (-1800 cal/mol), indicating that Ins(3,4,5,6)P₄ provokes a different or less extended conformational change of antithrombin than the pentasaccharide does.

When Ins(3,4,5,6)P₄ binds to antithrombin, Ins(3,4,5,6)P₄ does not induce conformational instability of antithrombin. In a preferred embodiment, the transformation to polymer or latent conformation by incubation of antithrombin at 65 °C or 42 °C is not modified by the presence of Ins(3,4,5,6)P₄.

The antithrombin activity can be measured using any commercially available kits evaluating anti-FXa or anti-lia activities by chromogenic or fluorogenic methods. On the other hand, Ins(3,4,5,6)P₄ does not affect the activity of factor X or thrombin.

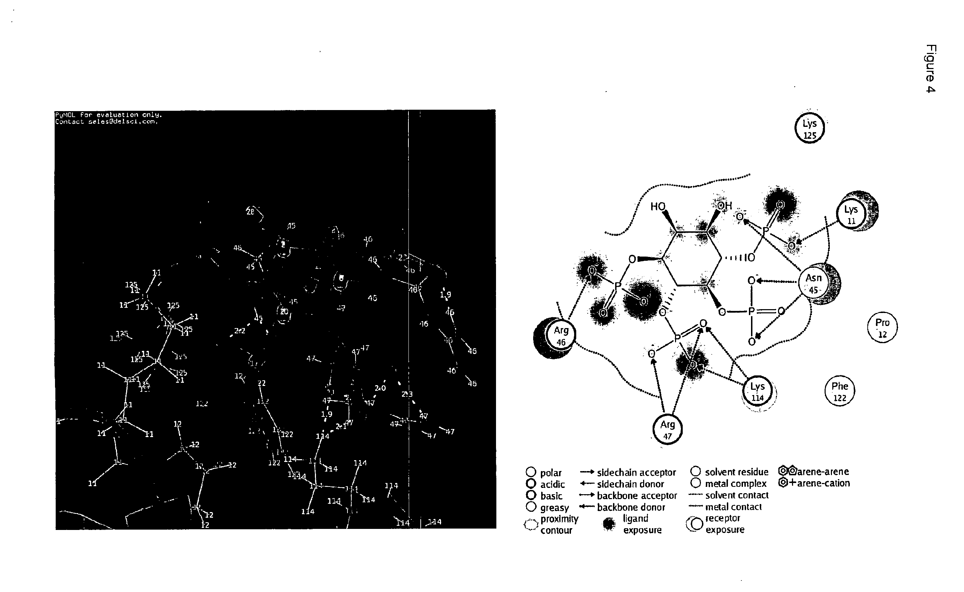

The computational analysis of Ins(3,4,5,6)P₄ and antithrombin shows that Ins(3,4,5,6)P₄ might interact with antithrombin through Arg residues of the heparin binding site of antithrombin (Fig. 4). Ins(3,4,5,6)P₄ can be purchased from several commercial vendors, which does not necessitate its chemical synthesis. Ins(3,4,5,6)P₄ shows a partial recovery of the antithrombin activity in patients with AT deficiency, where previous studies failed to manifest anticoagulant activity.

The Figures show:

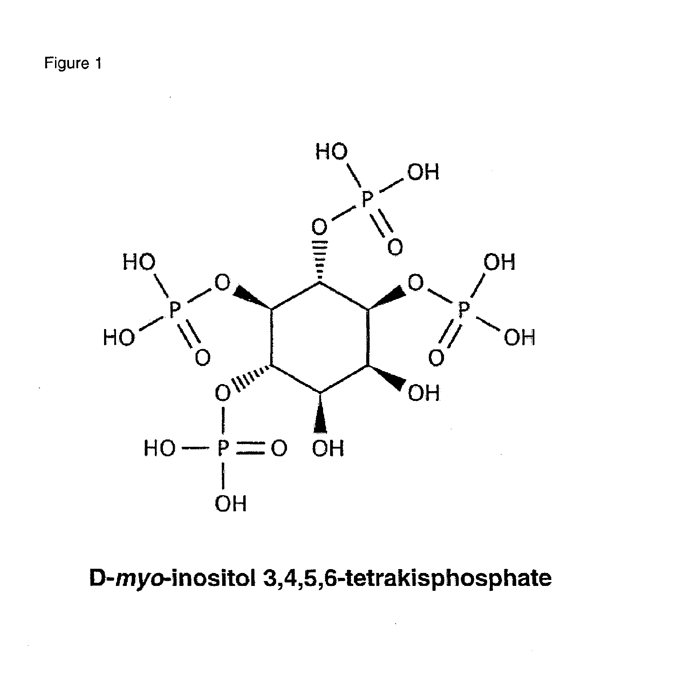

Figure 1: Structure of D-*myo*-inositol 3,4,5,6-tetrakisphosphate (Ins(3,4,5,6)P₄).
Figure 2: Isothermal titration calorimetry (ITC) profiles after titration of pentasaccharide (A), Ins(3,4,5,6)P₄ (B) and pentascharide at saturating concentration of Ins(3,4,5,6)P₄ (C) on a solution of antithrombin.
Figure 3: Fluorescence emission of antithrombin at 340nm. (A) Fluorescence intensity during titration of UFH (positive control). (B) Same as (A) but using polymers of antithrombin (negative control). (C) Effect of Ins(3,4,5,6)P₄ titration on the fluorescence emission of antithrombin. (D) Fluorescence emission of antithrombin during UFH titration in the presence of Ins(3,4,5,6)P₄. Fractional fluorescence is a means of at least 50 measurements.
Figure 4: Computational analysis of interaction between Ins(3,4,5,6)P₄ and antithrombin.

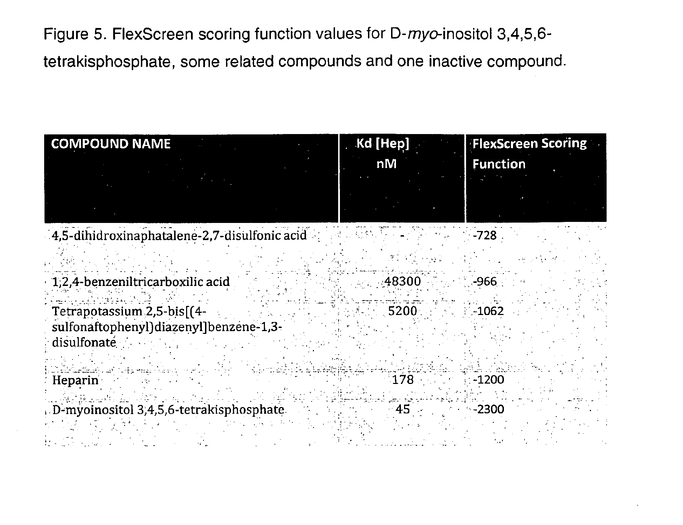

Figure 5: Flex Screen scoring function values for D-myo-inositol 3,4,5,6-tetrakisphosphate, some related compounds and one inactive compound.

The present invention will now be further illustrated in the following examples without being limited thereto.

### Examples

Abbreviations: AT, antithrombin; Ins(3,4,5,6)P₄, D-*myo*-inositol 3,4,5,6-tetrakisphosphate; Arg, arginine; UFH, unfractionated heparin: LMWH, low molecular weight heparin; PAD, peptidylarginine deiminase; EDTA, ethylenediamine tetraacetate; FXa, activated factor X; ITC, isothermal calorimetry.

Chromogenic substrates used in the Examples: S-2238, H-D-Phenylalanyl-L-pipecolyl-L-arginine-p-nitroaniline dihydrochloride (H-D-Phe-Pip-Arg-pNA·2HCl); S-2765, N-α-Benzyloxycarbonyl-D-arginyl-L-glycyl-L-arginine-p-nitroaniline-dihydrochloride (Z-D-Arg-Gly-Arg-pNA·2HCl).

Unfractionated heparin (UFH) is a highly sulfated glycosaminoglycan having molecular weight of 5000 to over 40,000 Da. The terms heparin and unfractionated heparin (UFH) described herein can be used interchangeably. Low molecular weight heparin (LMWH) is a heparin having average molecular weight of less than 8000 Da. Pentasaccharide fondaparinux is used as an anticoagulant for the inhibition of FXa. The pentasaccharide structure of fondaparinux is found in heparin, and is known to form a high affinity binding site for antithrombin.

### Example 1

Determination of the interaction of Ins(3,4,5,6)P₄ with antithrombin (AT) and calculation of the affinity by isothermal titration calorimetry (ITC).

The interaction of antithrombin (AT) with Ins(3,4,5,6)P₄ was evaluated by using isothermal titration calorimetry (ITC). With this method, protein is maintained in a cell where a compound is titrated by sequential injection. The interaction between both molecules is an exothermic reaction which accounts with a more negative enthalpy. The interaction between AT and Ins(3,4,5,6)P₄ was evaluated in comparison to that produced between AT and pentasaccharide fondaparinux (Arixtra®), hereinafter called pentasaccharide. Interaction between AT and pentasaccharide was also evaluated in the presence of saturating amount of Ins(3,4,5,6)P₄.

From this experiment, an affinity constant of 273 nM was calculated for the pentasaccharide. This affinity constant was 45 nM for Ins(3,4,5,6)P₄. However, the affinity constant for pentasaccharide in the presence of saturating concentration of Ins(3,4,5,6)P₄ was 18.8 nM.

From this result we can conclude that the presence of Ins(3,4,5,6)P₄ increases the affinity of antithrombin to the pentasaccharide ca. 14.5 fold.

### Method

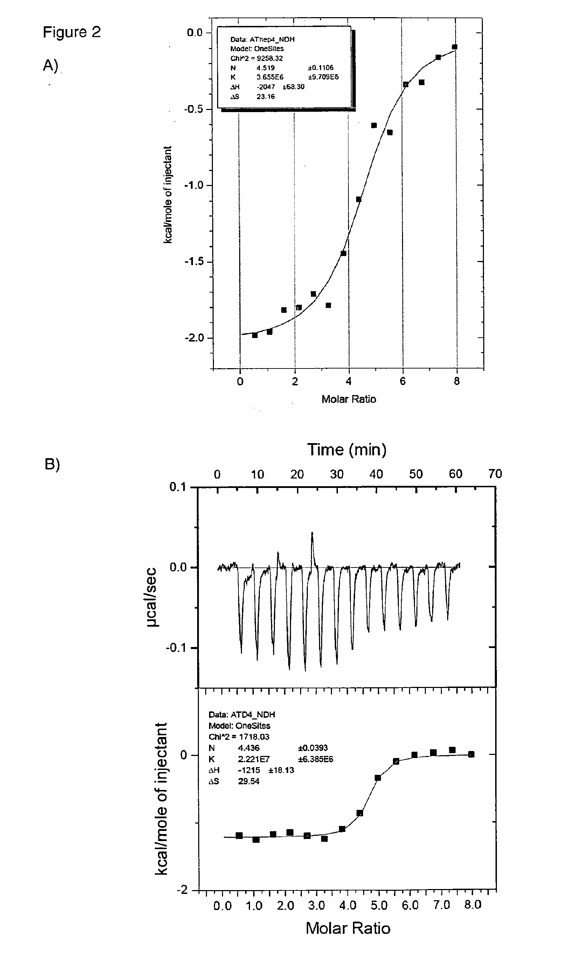

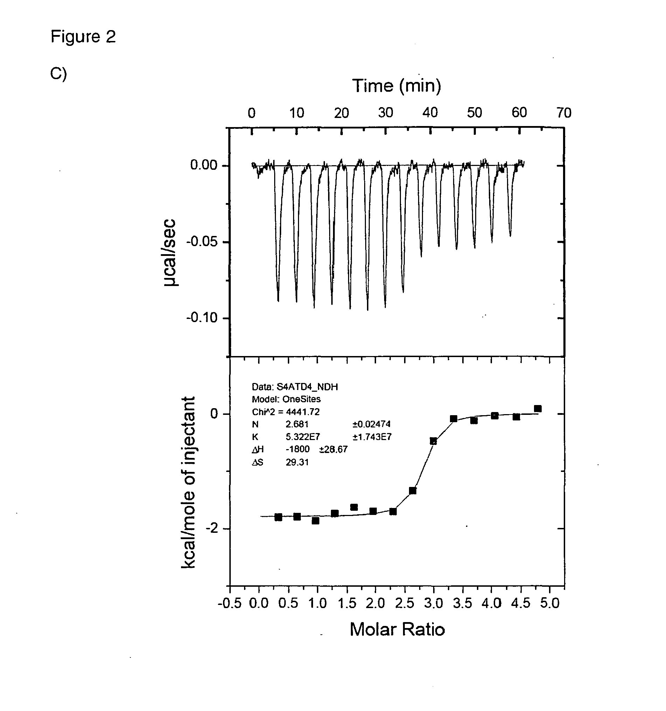

1.5 mL of AT (2.86 µM) in Tris-HCl pH 7.4 is added to the cell unit. The syringe is filled with 64 µM of pentasaccharide in order to carry out 15 injections of 20 µL (Fig. 2A). For the analysis of the binding of Ins(3,4,5,6)P₄, the same experiment was performed but 128 µM of Ins(3,4,5,6)P₄ was added to the syringe instead of pentasaccharide (Fig. 2B). For the analysis of the binding of pentasaccharide in the presence of Ins(3,4,5,6)P₄, the same experiment was performed but in this case AT (2.86 µM) and 128 µM of Ins(3,4,5,6)P₄ in Tris-HCl pH 7.4 are added to the cell unit. As the first case, titration was done with 64 µM of pentasaccharide (Fig. 2C).

### Example 2

Effect of Ins(3,4,5,6)P₄ on the activity of antithrombin and thrombin.

The anticoagulant activity of AT was determined by evaluating the inhibition of thrombin by purified or plasma AT using a chromogenic method. Thrombin hydrolyzes the chromogenic substrate S-2238 producing p-nitroaniline, whose emission can be measured at 405 nM. Thrombin and antithrombin are known to interact forming a covalent complex that inhibits the ability of thrombin to hydrolyze its substrate (Olson ST, Björk I. Predominant contribution of surface approximation to the mechanism of heparin acceleration of the antithrombin-thrombin reaction. Elucidation from salt concentration effects. J Biol Chem. 1991; 266(10): 6353-64). Antithrombin interacts with thrombin to form a covalent complex that inhibits the ability of thrombin to hydrolyze the chromogenic substrate. Thus no color was observed along the reaction. The presence of heparin accelerates the inhibition of thrombin by activation of antithrombin.

As a negative control, a mixture containing thrombin, antithrombin, and the substrate was measured, without any cofactor (Table1). As a positive control, a mixture containing thrombin, antithrombin, the substrate and low molecular weight heparin (LMWH) as a cofactor of antithrombin was measured (Table 1).

We also evaluated the potential direct effect of Ins(3,4,5,6)P₄ on thrombin activity. As indicated in Table 1, Ins(3,4,5,6)P₄ has no direct effect on thrombin activity.

### Method:

### Ex. 2-1. Measurement of the inhibition of thrombin by antithrombin:

75.1 µL Buffer (20 mM Tris-HCl, 150 mM NaCl, pH 7.4)
3.9 µL Antithrombin (12.9 µM)
1 µL Cofactor (LMWH (3.3 mM), Ins(3,4,5,6)P₄ (1.48 mM), or H₂O
10 µL Thrombin (47.6 nM)
10 µL Chromogenic substrate (S-2238) (1 mM)

### Ex. 2-2. Measurement of thrombin activity:

79 µL Buffer (20 mM Tris-HCl, 150 mM NaCl, pH 7.4)
1 µL Cofactor (LMWH (3.3 mM), Ins(3,4,5,6)P₄ (1.48 mM), or H₂O
10 µL Thrombin (47.6 nM)
10 µL Chromogenic substrate (S-2238) (1 mM)

All components except S-2238 were mixed and incubated at 37 °C for 5 min. S-2238 was then added and after 20 min, absorbance was measured at 405 nm in a plate reader (Synergy HT, UK).

**Table 1. Effect of Ins(3,4,5,6)P₄ on the activity of antithrombin and thrombin. 100% inhibition is established for the activity of thrombin in the presence of antithrombin activated by unfractionated heparin. From these results we can conclude that Ins(3,4,5,6)P₄ is able to provoke the inhibition of thrombin without affecting directly the activity of thrombin.**

| | Ex. 2-1 | Ex. 2-2 |
|---|---|---|
| | % Inhibition of thrombin by antithrombin | % Thrombin activity |
| LMWH | 94.84 ± 1.03 | 93.86 ± 1.22 |
| Ins(3,4,5,6)P₄ | 21.92 ± 2.06 | 100.0 ± 1.43 |
| No cofactor | 9.250 ± 0.98 | 93.81 ± 1.13 |

### Example 3

Fluorescence studies to determine the potential conformational activating effect of Ins(3,4,5,6)P₄

There are four tryptophanes in antithrombin that are excited at 280 nm and produce the emission of fluorescence at 340 nm (W49, 189, 225, and 307). This intrinsic fluorescence increases when antithrombin is activated by heparin due to the conformational changes in the molecule. As a consequence, the affinity of antithrombin for the heparin can be calculated indirectly by measuring the emission of fluorescence at different concentrations of heparin until the affinity reaches saturation.

### Method:

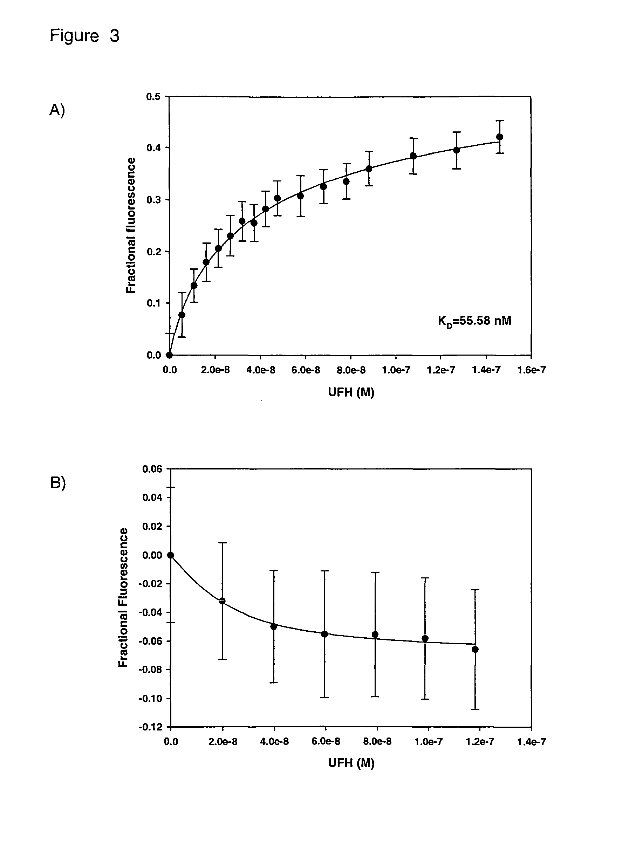

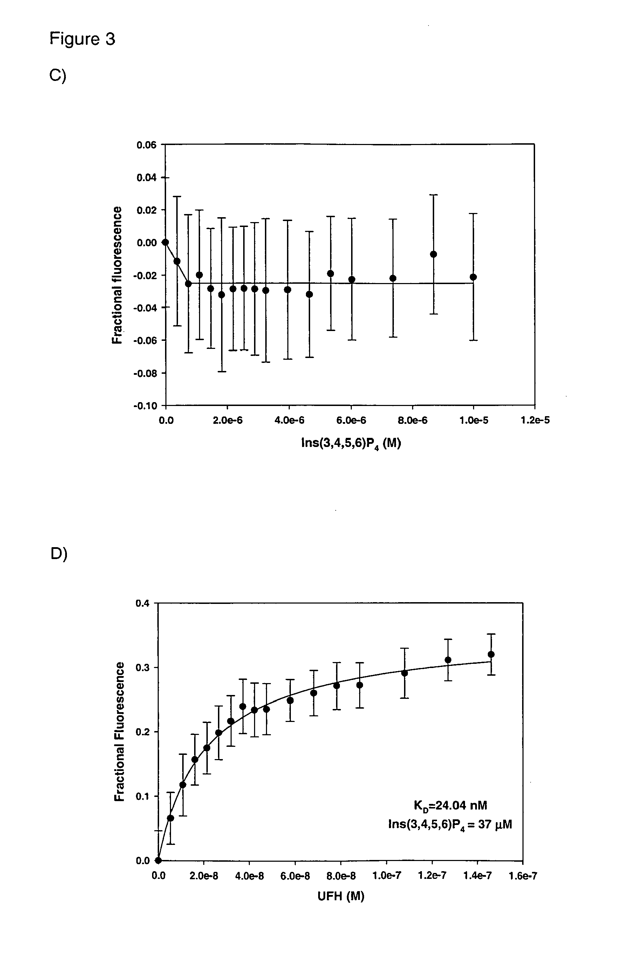

(1) As a positive control, the fluorescence intensity of AT (25 nM) was measured in the presence of unfractionated heparin (UFH) (Fig. 3A). As shown in Fig. 3A, AT was found to exhibit an affinity for UFH of K_{D} = 55.58 nM. Binding of UFH to AT triggers a conformational change in AT which increases the fractional fluorescence of AT.
(2) As a negative control, the fluorescence of AT polymer (25 nM) was measured in the presence of unfractionated heparin (UFH) (Fig. 3B). Addition of UFH does not affect the fractional fluorescence of AT polymer.
(3) The experiment was performed according to above (1), except that different concentrations of Ins(3,4,5,6)P₄ were added instead of UFH (Fig. 3C). As shown in Fig 2C Ins(3,4,5,6)P₄ caused negligible changes in the endogenous fluorescence of AT.
(4) The experiment was performed according to above (1), except that a mixture of AT (25 nM) and Ins(3,4,5,6)P₄ (37 µM) was used instead of only AT (Fig. 3D). Ins(3,4,5,6)P₄ was found to increase the affinity of antithrombin to heparin by 2.3 fold: From K_{D}= 55.58 nM to K_{D}= 24.04 nM (Fig. 3A and Fig. 3D).

From these results, it is shown that Ins(3,4,5,6)P₄ does not induce the same conformational activation of antithrombin as heparin, but increases the binding affinity of antithrombin to heparin.

### Example 4

Partial activation of antithrombin by Ins(3,4,5,6)P₄ was examined.

Cirtrullination of antithrombin was performed as described (A. Ordóñez *et al.* FEBS J. 2009, 276(22), 6763-72) in the presence of heparin or Ins(3,4,5,6)P₄ as well as without these molecules. Arg residues of anithrombin are converted to citrullines in the presence of peptidylarginine deiminase (PAD). When Arg393 (P1) of antithrombin is converted to citrulline, antithrombin loses its function. The loss of function provoked by citrullination is accelerated by heparin, for instance low molecular weight heparin (LMWH), since the P1 residue becomes more accessible by the activation induced by heparin. Both, heparin and Ins(3,4,5,6)P₄ induced a rapid (15 min) citrullination of Arg393 (P1) that caused the loss of function, supporting that these molecules caused the exposure of the P1 residue (Table 4).

### Method:

The reaction mixture of a total volume of 10 µL consisting of the following components (1) to (4) was incubated at 37 °C for 15 min before the reaction was stopped with EDTA (10 µL, 100 mM).
(1) 3.6 µL AT (129 µM)
(2) 1 µL buffer (1 M Tris-HCl - 50 mM CaCl₂, pH 7.4)
(3) 1 µL PAD (0.488 mg/mL)
(4) 4.4 µL H₂O or 1 µL Ins(3,4,5,6)P₄ (1.48 mM) + 3.4 µL H₂O or 1 µL LMWH (1.4 mM) + 3.4 µL H₂O

A mixture of 30 µL citrullinated sample (pH 7.4, 15.48 nM) and 30 µL FXa (16.28 nM) was incubated in the presence of 20 mM Tris-HCl, 150 mM NaCl at 37 °C for 3 min and then 30 µL chromogenic substrate S-2765 (0.3 mM) was added. The reaction was performed in a 96-well plate. The absorbance at 405 nm was measured at 0, 3, and 6 minutes. Table 2 shows absorbance at 6 min after the start of the cirtullination reaction.

Conclusion: Ins(3,4,5,6)P₄ should provoke the exposition of P1

**Table 2. Effect of Ins(3,4,5,6)P₄ on the citrullination of R393 in antithrombin by Peptidylarginine deiminase in comparison to Low Molecular Weight Heparin. 100% antithrombin activity is established for the antithrombin activity obtained in the absence of any other cofactor or protease in its inhibition of the target FXa. From these results we can conclude that Ins(3,4,5,6)P₄ is able to provoke the exposition of the reactive centre loop, thus facilitating the action of PAD, as occurred with any type of heparin.**

| | **% AT Activity** |
|---|---|
| AT | 100 ± 0.5 |
| AT + PAD | 99.4 ± 1.2 |
| AT + PAD + LMWH | 30.0 ± 0.3 |
| AT + PAD + Ins(3,4,5,6)P₄ | 30.0 ± 0.5 |

### Example 5

Conformational stability of antithrombin in the presence of Ins(3,4,5,6)P₄ was tested.

Incubation of AT with Ins(3,4,5,6)P₄ does not induce conformational instability. The transformation to polymer or latent conformation by incubation of AT at 65°C or 42°C is not modified by the presence of Ins(3,4,5,6)P₄.

### Example 6

Partial recovery of anticoagulant activity in patients with type II antithrombin deficiency was tested.

The antithrombin activity of plasma from a patient homozygous for the L99F mutation (AT Budapest III -L99F-), as well as plasma from a patient having heterozygous mutations affecting the heparin binding site, i.e. AT Toyama -R47C- or Basel -P41 L-, was studied. Using a chromogenic method, Ins(3,4,5,6)P₄ induced a 19% activation of AT by heparin (Table 3) Similar activation (11 %) was achieved in a system that used endothelial cells expressing heparan sulphate (Table 4), where no heparin is added. However, no significant activation was observed when plasma of patients with heterozygous mutations affecting the heparin binding site (AT Toyama -R47C- or Basel -P41 L-) was used. These results may be explained by the residues of AT involved in the interaction with Ins(3,4,5,6)P₄ (Fig. 4).

### Method:

To 30 µL of plasma containing AT Budapest, AT Toyama, or AT Basel with or without 2 µL Ins(3,4,5,6)P₄ (148 µM), 33 µL Thrombin (8.73 nM) was added and the mixture was incubated for 5 min at 37°C in the presence of UFH (1.27 mM). 20.9 µL S-2238 (1 mM) was added and after 20 min, absorbance was measured at 405 nm (Table 3).

**Table 3. Effect of Ins(3,4,5,6)P₄ on the activation of antithrombin by heparin in different antithrombin deficiency plasma samples. 100% antithrombin activity is established for the antithrombin activity obtained in the presence of unfractionated heparin in its inhibition of thrombin. From these results we can conclude that Ins(3,4,5,6)P₄ is able to provoke a higher inhibition of thrombin.**

| **Plasma** | **Ins(3,4,5,6)P₄** | **% AT Activity** |
|---|---|---|
| Control | - | 97.00 ± 3.77 |
| Control | + | 97.34 ± 2.70 |
| Budapest | - | 33.22 ± 2.93 |
| Budapest | + | 40.05 ± 4.19 |
| Toyama | - | 52.57 ± 4.43 |
| Toyama | + | 42.55 ± 1.98 |
| Basel | - | 67.80 ± 3.27 |
| Basel | + | 54.20 ± 1.81 |

### Method:

To confluent endothelial cells previously fixed on 96-well plates with 4% formaldehyde, 30 µL of plasma containing AT Budapest, AT Toyama, or AT Basel with or without 2 µL Ins(3,4,5,6)P₄ (148 µL), 33 µL Thrombin (8.73 nM) was added and the mixture was incubated for 5 min at 37 °C. 20.9 µL S-2238 (1 mM) was added and after 20 min, absorbance was measured at 405 nm (Table 4).

**Table 4. Effect of Ins(3,4,5,6)P₄ on the activation of antithrombin by endothelial heparan sulfates in Budapest III antithrombin deficiency plasma. 100% antithrombin activity is established for the antithrombin activity obtained in the absence of any cofactor but after binding to the endothelial heparin sulphates in the inhibition of thrombin. From these results we can conclude that Ins(3,4,5,6)P₄ is able to provoke a higher inhibition of thrombin.**

| **Plasma** | **Ins(3,4,5,6)P₄** | **% AT Activity** |
|---|---|---|
| Control | - | 100.0 ± 0.2 |
| Control | + | 101.4 ± 0.1 |
| Budapest | - | 33.2 ± 2.9 |
| Budapest | + | 37.2 ± 0.8 |

## Claims

1. D-*myo*-inositol 3,4,5,6-tetrakisphosphate for use in activating antithrombin in a patient.

2. D-*myo*-inositol 3,4,5,6-tetrakisphosphate for use according to claim 1, wherein the patient is suffering from a disease associated with increased blood clotting.

3. D-*myo*-inositol 3,4,5,6-tetrakisphosphate for use according to claim 2, wherein the disease associated with increased blood clotting is a thromboembolic disease.

4. D-*myo*-inositol 3,4,5,6-tetrakisphosphate for use according to claim 3, wherein thromboembolic disease is selected from the group consisting of arterial thrombosis, stroke, and myocardial infarction.

5. D-*myo*-inositol 3,4,5,6-tetrakisphosphate for use according to any of claims 1 to 4, wherein the administration of D-*myo*-inositol 3,4,5,6-tetrakisphosphate is a prophylaxis for a disease associated with increased blood clotting.

6. D-*myo*-inositol 3,4,5,6-tetrakisphosphate for use according to any of claims 1 to 4 for use in recovery of antithrombin activity in patients with antithrombin deficiency.

7. Pharmaceutical composition containing D-*myo*-inositol 3,4,5,6-tetrakisphosphate for use in recovery of antithrombin activity in patients with antithrombin deficiency.

8. Pharmaceutical composition containing D-*myo*-inositol 3,4,5,6-tetrakisphosphate for use in prophylaxis of a disease associated with increased blood clotting.
